# EUROPEAN PATENT APPLICATION

(11) **EP 4 582 020 A1**
(43) Date of publication of application: **09.07.2025**
(21) Application number: 24382010.7
(22) Date of filing: 08.01.2024
(51) Int. Cl.: A61B 5/145, A61B 5/1486, A61B 5/00

(54) **MICRONEEDLE BIOSENSOR AND REMOTE MONITORING SYSTEM FOR REAL-TIME HORMONE TREATMENT**

(71) Applicant: Universitat D'Alacant / Universidad de Alicante, 03690 San Vicente del Raspeig-Alicante (ES); Technische Universität Dresden Körperschaft des öffentlichen Rechts, 01069 Dresden (DE)
(72) Inventor: GORELOVA, Anastasiia, 03690 San Vicente del Raspeig (ES); MELIÁ BEIGBEDER, Santiago, 03690 San Vicente del Raspeig (ES); PARICHENKO, Alexandra, 01069 Dresden (DE); IBARLUCEA, Bergoi, 01069 Dresden (DE); CUNIBERTI, Gianaurelio, 01069 Dresden (DE); MEDINA SÁNCHEZ, Mariana, 01069 Dresden (DE)
(74) Representative: TRBL Intellectual Property

(57) **Abstract**

The present invention relates to a biosensor (1) for the measurement of chemical substances (2) in biological samples, applicable in clinical diagnosis, comprising mainly a substrate matrix (3), a plurality of hollow microneedles (4) and a sensing unit (6) based on a plurality of bioelectrodes (611). The biosensor (1) is characterized in that the sensing unit (6) comprises a plurality of valves (62) adapted to control the passage of biological samples from the hollow microneedle (4) to a sensing chamber (61). It additionally relates to a system (9) for monitoring biological samples applicable in clinical diagnosis comprising said biosensor (1) and to a method for measuring and monitoring the chemical substances (2) by using said system (9).

## Description

### FIELD OF THE INVENTION

The present invention belongs to the technical field of biotechnology and computer science. More specifically, the invention relates to a biosensor for identification of chemical substances in biological samples. The invention is preferably used in clinical diagnostics and hormone treatment.

### BACKGROUND OF THE INVENTION

Remote patient monitoring (RPM) relates to a broad variety of technologies and devices that enable monitoring and treatment of patients outside of conventional clinical settings, such as in the home or in a remote area, thereby increasing access to care services and treatments, as well as decreasing healthcare delivery costs. RPM typically involves the continuous monitoring of patients during long periods of time, in order to track physical symptoms, chronic conditions, or post-hospitalization rehabilitation with improved accuracy. However, despite continuous medical and technological advances, nowadays there are still many treatments that have not been addressed with RPM techniques, due to the absence of reliable devices and smart health monitoring systems (SHMS) that provide accurate real-time information on particular substances or, in general, specific clinical indicators. This is the case for hormones, which are chemical messengers with high potency that travel through the bloodstream, and that are responsible for growth and development, metabolism, fertility, sex life and mood, among other functions. Due to their effect in the organism, it is of uttermost importance to have an adequate hormonal balance, since a small number of hormones can cause major changes in cells, and even in the whole body. In fact, hormone deficiency or excess is linked to diseases with higher mortality and lower birth rates, because of their link to fertility.

The main causes of death in the European Union are related to cardiovascular diseases and cancer. In both cases, there are several studies that indicate their strong relationship with hormonal balance and, therefore, the need to develop a better hormonal control in the treatments of these diseases. Infertility is another relevant condition which affects the European population, which in recent years has increased to very worrying levels. Recent studies indicate that one in six couples have fertility problems, which in most cases are related to imbalances in the sex hormones of both parents. All infertility treatment methods involve hormone therapy and therefore, in order to avoid side effects, the level of hormones injected needs to be precisely controlled.

Recent studies in the EU point out to the need to invest in obtaining more scientific evidence of the efficacy of SHMS by monitoring large-scale experiments. For this reason, it is essential to reduce the economic and time costs of conducting such experiments with real data. In this respect, it is recognized that SHMS should be complemented by modelling and simulation techniques, so as to predict a complete scenario of specific treatments, which would allow decisions to be made on the basis of synthetic simulation data, thus reducing the number of clinical trials on real patients.

Recently, a novel paradigm called Digital Twin (DT) has emerged with great potential, which allows the representation of virtual entities that are not limited only to the devices of a SHMS, but allow the full representation of the real objects that make up the scenario, e.g., patients, processes, tasks and devices. The use of simulation would accelerate the collection of data before the scenario has been implemented and would also allow first feedback from healthcare experts on the solution.

Therefore, there is a need to improve the treatment of hormonal pathologies, achieving a remote monitoring of hormone levels over a certain period of time and data that can be used by automatic processing techniques, such as artificial intelligence or machine learning techniques, to predict patterns in said pathologies and their treatments, and thereby helping healthcare experts to make decisions on possible actions to be taken in the medical prescription of these diseases.

### BRIEF DESCRIPTION OF THE INVENTION

A first object of the present invention relates to a biosensor for measuring chemical substances in biological samples, applicable in clinical diagnosis. The biosensor preferably comprises:
- a substrate matrix;
- a plurality of hollow microneedles, adapted for penetrating a subject's skin or tissue and obtaining a biological sample, wherein each of the hollow microneedles comprises a first end and a second end, wherein the first end is adapted to penetrate the subject's skin or tissue, and wherein the second end is at least partially embedded in the substrate matrix;

- a sensing unit, adapted for measuring information related to one or more chemical substances in the biological sample through a potential variation, wherein the sensing unit is arranged on the substrate matrix and comprises one or more sensing chambers, wherein each of said sensing chambers further comprises;
   - a plurality of bioelectrodes, adapted for detecting the chemical substances, wherein the plurality of bioelectrodes comprise a selective membrane to the chemical substances of the biological sample;
   - an electronic circuit adapted for applying an electric current between the bioelectrodes;
      and wherein the potential variation takes place when one or more chemical substances in the biological sample bind to the selective membrane of the bioelectrodes; and
- a microcontroller, adapted for storing the information related to the chemical substances in the biological sample.

According to the above first object, the invention allows measuring chemical substances in biological samples in a minimally invasive way, due to the micro size of the hollow microneedles. Moreover, and advantageously in the invention, the sensing unit comprises a plurality of valves adapted for controlling the passage of the biological sample from the first end of the hollow microneedle into the sensing chamber.

In a preferred embodiment of the invention, the bioelectrodes comprise a semiconductor nanomaterial coupled with a plurality of electrical sensors. The nanoscopic dimension of the semiconductor material allows a high sensitivity to chemical substances.

In another preferred embodiment of the invention, the electrical sensors are selective from: field effect transistors, electrochemical sensors or impedimetric sensors.

In another preferred embodiment of the invention, the field effect transistors are embedded in one or more flexible polymer layers; and/or the semiconductor nanomaterial comprises carbon nanotubes, graphene, transition metal dichalcogenide monolayers or silicon nanowires. Using flexible polymer layers allows achieving a lightweight and portable biosensor. In addition, the use of polymer layers allows the sensing unit to be used as low-cost disposable product that can be replaced after several measurements. By being able to replace only the sensing unit, it is also possible to extend the lifetime of the biosensor and consequently save costs. It also improves the subject's quality of life, as the biosensor needs to be replaced less frequently.

In another preferred embodiment of the invention, the selective membranes to the chemical substances of the biological sample are based on polymers and biomolecules with high affinity for biological samples, wherein the polymers further comprise a dielectric polymer and biomolecules comprise aptamers, antibodies, nanobodies and/or enzymes.

In another preferred embodiment of the invention, the hollow microneedles comprise photoresist based on hydrogels or conductive polymers.

In another preferred embodiment of the invention, the valves comprise polypyrrole actuators.

In another preferred embodiment of the invention, the biosensor is embedded in an adhesive patch. Thus, the subject does not have to prick himself every time he wants to take a measurement, thereby improving user's quality of life. Moreover, since the patch is in continuous contact with the subject's skin or tissue from which the biological sample is taken, it is possible to carry out continuous chemical substances' measurements.

In another preferred embodiment of the invention, the chemical substances comprise hormones.

In another preferred embodiment of the invention, the biosensor further comprises a drug reservoir with an opening controlled by delivery means, wherein the delivery means is adapted for delivering a drug through the opening, preferably as a function of the concentration of the chemical substances present in the biological sample, as measured by the sensing unit or as read from the information derived from said concentration. This allows the delivery of drugs with improved accuracy according to the subject's clinical needs.

In another preferred embodiment of the invention, the electronic circuit is powered by a battery or a wireless power system. Thus, the electronic circuit has improved energy autonomy, and does not need to be connected to a grid, thereby allowing the biosensor for improved portability.

A second object of the invention relates to a system for monitoring biological samples, preferably applicable in clinical diagnosis, comprising:
- a biosensor according to any of the embodiments described in the present document, wherein the microcontroller further comprises communication means adapted for transferring the stored information related to the chemical substances in biological samples to a monitoring unit;
- a monitoring unit, further comprising;
   - processing means, configured for processing the transferred information related to the chemical substances in biological samples by the communication means and generating alerts based on this information;
   - display means, adapted for displaying the processed information related to the chemical substances in biological samples.

In another preferred embodiment of the invention, the communication means is based on Bluetooth, WiFi, NFC and/or 5G protocols, or on a combination thereof. Thanks to this, it is not necessary to connect the biosensor directly with a cable to the monitoring unit to transfer the stored information. This allows the subject wearing the biosensor and the medical specialist to be in different locations, thereby allowing the remote display of the processed information and associated alerts remotely.

In yet another preferred embodiment of the invention, the monitoring unit comprises a mobile device, such as a smartphone or a tablet. This allows the subject or the medical specialist to visualize the monitoring at any time, since the mobile device is portable and can be easily carried.

In another preferred embodiment of the invention, the processing means comprises a Scenario Modelling framework and an artificial intelligence platform.

A third object of the invention relates to a method for measuring and monitoring chemical substances in biological samples by using a system according to any one of the previously described embodiments, wherein the method comprises the following steps:
a) placing the biosensor on a subject's skin or tissue;
b) taking biological samples with the first end of the hollow microneedles;
c) applying an electric current between the bioelectrodes with the electronic circuit;
d) measuring the information related to the chemical substances in biological samples with the sensing unit;
e) storing the measured information related to the chemical substances in biological samples with the microcontroller;
f) transferring the measuring information related to the chemical substances in biological samples with the communication means from the biosensor to the monitoring system;
g) processing the transferred information related to the chemical substances in biological samples with the display means and generating alerts based on this information; and
h) displaying the alerts on the monitoring unit.

### BRIEF DESCRIPTION OF THE DRAWINGS

The above detailed and other features and advantages of the present invention will become apparent in view of the following non-limiting description of different embodiments of the invention, made with reference to the accompanying drawings, where:
Figure 1 shows a schematic representation of the biosensor according to an embodiment of the invention in the context of a preferred application of said biosensor for measuring chemical substances in biological samples.
Figure 2 shows the sensing chambers of a biosensor according to an embodiment of the invention.
Figure 3 shows a bioelectrode of a biosensor according to an embodiment of the invention, wherein the bioelectrode comprises a field effect transistor with three terminals (source, drain and gate), a plurality of carbon nanotubes which are aligned with the channel of the field effect transistor, and which comprise a selective membrane for biorecognition.
Figure 4 shows a device according to an embodiment of the invention applied to the measure and monitor of chemical substances in biological samples by placing one biosensor on a subject's skin or tissue.

### NUMERICAL REFERENCES USED IN THE DRAWINGS

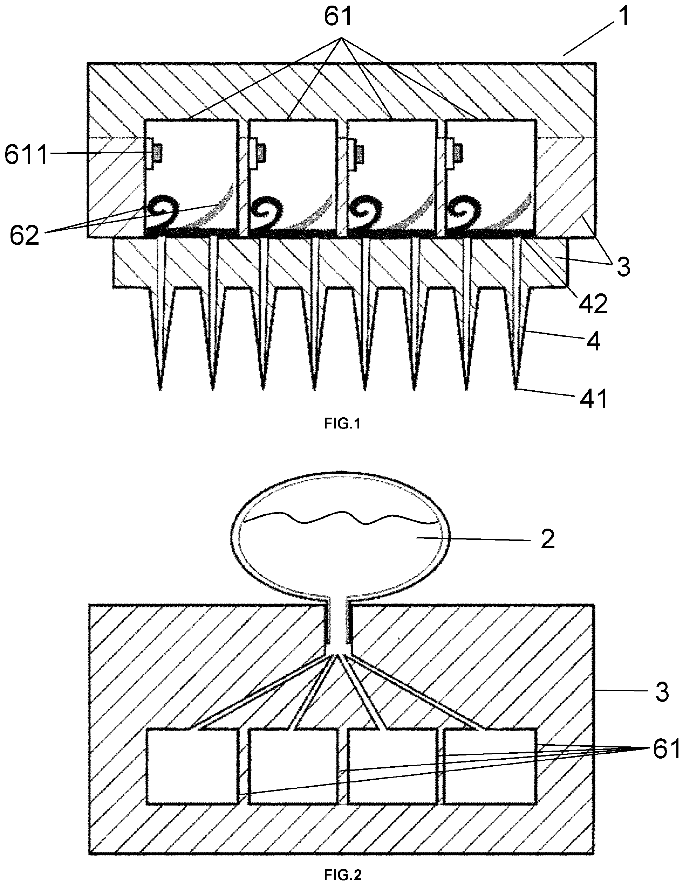

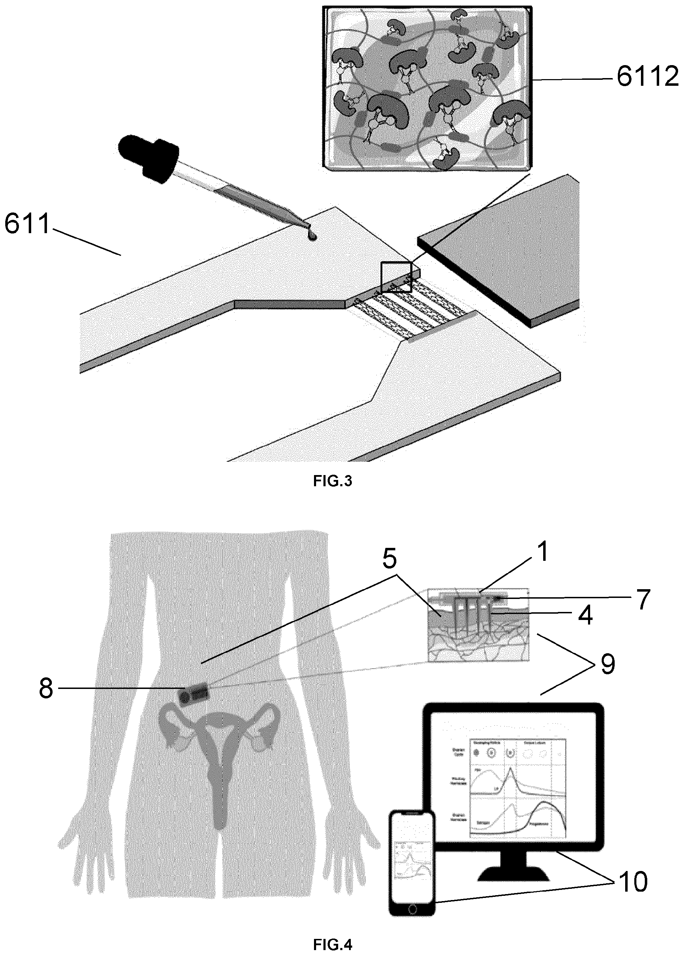

In order to provide a better understanding of the technical features of the invention, the referred Figures 1-4 are accompanied of a series of numerical references which, with an illustrative and non-limiting character, are hereby represented:

| | |
|---|---|
| 1 | Biosensor |
| 2 | Chemical substances |
| 3 | Substrate matrix |
| 4 | Hollow microneedles |
| 41 | First end of the hollow microneedle |
| 42 | Second end of the hollow microneedle |
| 5 | Subject's skin or tissue |
| 6 | Sensing unit |
| 61 | Sensing chambers |
| 611 | Bioelectrodes |
| 6112 | Selective membranes |
| 612 | Electronic circuit |
| 62 | Valves |
| 7 | Microcontroller |
| 8 | Adhesive patch |
| 9 | System for monitoring biological samples |
| 10 | Monitoring unit |

### DETAILED DESCRIPTION OF THE INVENTION

As described in precedent sections, the present invention proposes a biosensor (1) for measuring chemical substances (2) in biological samples, applicable in clinical diagnosis. Under the scope of interpretation of the present invention, the expression "biological sample" will be understood as a sample obtained from the skin or tissue of a biological subject, whether of animal or human origin. Thus, a subject will be understood to be any animal or human from which biological samples are extracted. In a preferred embodiment of the invention, the biosensor (1) comprises a substrate matrix (3), a plurality of hollow microneedles (4), a sensing unit (6) and a microcontroller (7).

The substrate matrix (3) acts like a support for the rest of elements which comprise the biosensor (1). In a preferred embodiment of the invention, the substrate matrix (3) comprises polydimethylsiloxane polymer. This polymer exhibits excellent resistance to biodegradation, biocompatibility, chemical stability, gas permeability and good mechanical properties. For this reason, the polydimethylsiloxane polymer is a suitable material for manufacturing medical devices such as the biosensor (1). The plurality of hollow microneedles (4) can comprise a single hollow microneedle (4) or an array of hollow microneedles (4). The hollow microneedles (4) comprise a first end (41) and a second end (42), and are adapted for penetrating a subject's skin or tissue (5) and obtaining a biological sample from the subject's skin or tissue (5). For this purpose, the second end (42) of the microneedles (4) is at least partially embedded in the substrate matrix (3) for fixing them onto the substrate matrix (3), while their first end (41) is free. The first end (41) is not fixed to the substrate matrix (3) as it is adapted to penetrate the subject's skin or tissue (5). Once the first end (41) has penetrated on the subject's skin or tissue (5), the biological sample flows through the interior of the hollow microneedle (4) until it reaches the sensing unit (6), through capillarity. In a preferred embodiment of the invention, to boost the flow of the biological sample within the interior of the hollow microneedle (4), a pump or an electrophoretic flow can be used. The electrophoretic flow can be achieved by integrating electrodes and/or improving the capillary flow. For this purpose, the electrodes can be placed at different locations with respect to the hollow microneedles (4), such as between the hollow microneedles (4) or integrated inside the hollow microneedles (4) themselves. To improve the capillary flow, the surface of the hollow microneedle (4) can be modified, by applying hydrophilic coating, varying cross-sections or combining with porous sections that adsorb the biological sample. The micro size of the hollow microneedles (4) reduces the contact surface of the first end (41) with the subject's skin or tissue (5), so that in the damage caused to the subject's skin or tissue (5) is minimal. This makes the biosensor (1) minimally invasive for the subject. The sensing unit (6) is arranged on the substrate matrix (3) and adapted for measuring information related to one or more chemical substances (2) obtained from the biological sample. For that purpose, the sensing unit (6) comprises one or more sensing chambers (61), wherein each of said sensing chambers (61) further comprises a plurality of bioelectrodes (611) and an electronic circuit (612). The bioelectrodes (611) are a type of electrodes adapted for detecting the chemical substances (2), which comprise a selective membrane (6112) to the chemical substances (2) obtained from the biological samples. Thus, within the scope of the present invention, the expression bioelectrodes (611) can be understood as biosensors for detecting chemical substances (2). In the present invention, the expression "detecting the chemical substances (2)" can also be referred to as "biorecognition". The electronic circuit (612) is adapted for applying an electric current between the bioelectrodes (611) and measuring an electrical signal change, specifically a variation of electric potential. The variation of potential takes place when one or more chemical substances (2) of the biological samples bind to the selective membrane (6112) of the bioelectrodes (611). Under the scope of interpretation of the present invention, the expression 'selective membrane' (6112) is understood as a membrane functionalized with chemical molecules, wherein these chemical molecules bind specifically to the chemical substance (2) obtained from the biological sample. Thus, the measure of the information related to one or more chemical substances (2) measured by the sensing unit (6) is taken through the variation of potential. In the present invention, the variation of potential is proportional to the concentration of chemical substances (2) on the biological sample. Once the biorecognition is performed, the information related to one or more chemical substances (2) or, in other words, the chemical substance (2) concentration on the biological sample, is stored in the microcontroller (7). Advantageously, the sensing unit (6) comprises a plurality of valves (62) adapted for controlling the passage of the biological samples from the first end (41) of the hollow microneedle (4) into the sensing chamber (61). In order to control the biological sample passage, the valves (62) must be composed of any stimuli-responsive material, thus reacting to a stimulus. Thus, depending on the stimulus to which the valves (62) respond, the valves (62) can be physically actuated valves or can be composed with hydrophilic and/or hydrophobic sections. Within the physically actuated valves, the valves (62) can be, for example, mechanical, electrical, electromagnetic or thermal. Under the scope of interpretation of the present invention, the expression "passage" of the biological sample into the sensing chamber (61) can also be interpreted as the flow of the biological sample into the sensing chamber (61).

In another preferred embodiment of the invention, the bioelectrodes (611) comprise a semiconductor material coupled with a plurality of electrical sensors. Under the scope of the present invention, an electrical sensor is a sensor that detects a physical parameter of interest (i.e., chemical substances (2)) and converts it into an electrical signal that can be measured. In another preferred embodiment of the invention, electrical sensors are selective from electrochemical sensors, impedimetric sensors or field effect transistors. Under the scope of interpretation of the present invention, the field effect transistors are a type of transistors comprised by three terminals (source, drain and gate) and a channel through which an electrical current passes. In this embodiment, the semiconductor material is used for the chemical substances (2) biorecognition. For this purpose, the semiconductor material comprises the selective membrane (6112) to the chemical substances (2) obtained from the biological samples and is aligned with the channel of the field effect transistors by di-electrophoresis. In the present invention, the bioelectrodes (611) can comprise a single field effect transistor or an array of field effect transistors. Depending on the number of field effect transistors and thus the number of semiconductor materials in the bioelectrode (611), the bioelectrode (611) will measure one or more types of chemical substance (2). The bioelectrode (611) measures one type of chemical substance per one field effect transistor. Thus, if the bioelectrode (611) comprises several field effect transistors, several chemical substances (2) are measured with a single biosensor (1). Once the biological sample reaches the bioelectrode (611), the biorecognition is done by the selective membrane (6112) of the semiconductor material. And as it has been mentioned above, the biorecognition is translated into a potential change within the bioelectrode (611), which is measured by the electronic circuit (612). This potential change, thanks to the nanoscopic dimension of the semiconductor material, is measurable in real time and with high sensitivity.

In another preferred embodiment of the invention, the field effect transistors are embedded in one or more flexible polymer layers. Using flexible polymer layers allows the manufacture of lightweight and portable sensors. In addition, the use of polymer layers allows the sensing unit (6) to be used as low-cost disposable consumables that can be replaced after several measurements. Once the sensing unit (6) has been discarded, it can be replaced by another one. When the sensing unit (6) is replaced, neither the substrate matrix (3) nor the hollow microneedles (4), nor the microcontroller (7) need to be replaced, these parts remain in the biosensor (1). By being able to replace only the sensing unit (6), it is possible to extend the lifetime of the biosensor (1) and consequently save costs. It also improves the subject's quality of life, as the biosensor (1) needs to be replaced less frequently, reducing the number of pricks on the subject's skin or tissue (5). Under the scope of interpretation of the present invention, the expression "prick" refers to the action in which the subjects place the biosensor (1) on his skin or tissue, so that the first ends (41) of the hollow microneedles (4) penetrate the skin or tissue (5).

In a preferred embodiment of the invention, the semiconductor nanomaterials which are coupled to the field effect transistor can be composed of a semiconductor material with any dimensional structure, whether 0D, 1D, 2D or 3D. Preferably, the semiconductor nanomaterials comprise carbon nanotubes, graphene, transition metal dichalcogenide monolayers or silicon nanowires. To manufacture the field effect transistor with a semiconductor nanomaterial, the terminals (source, drain and gate) of the field effect transistor are preferably fabricated by photolithography. Then the semiconductor material is aligned with the channel of the field effect transistor by di-electrophoresis. In addition, the semiconductor nanomaterial comprises the selective membranes (6112) for biorecognition. For this, the semiconductor nanomaterial is functionalized with chemical molecules, wherein these chemical molecules bind specifically to the chemical substance (2) obtained from the biological sample. In a preferred embodiment of the invention, pyrene-based crosslinkers are used for chemical molecules immobilization on the semiconductor nanomaterial. Preferably, polyethylene glycol (PEG) co-immobilization is addressed using mPEG-pyrene or PEG-based hydrogels to increase the Debye length, thus improving the sensitivity of the biosensor (1).

In a preferred embodiment of the invention, the selective membranes to the chemical substances (2) of the biological samples are based on polymers and biomolecules with high affinity for these chemical substances (2). The biomolecules are attached to the bioelectrodes (611) to ensure the detection of the chemical molecules (2) and preferably comprise aptamers, antibodies, nanobodies and/or enzymes. The polymers are used to host the biomolecules on the surface of the semiconductor nanomaterial and to increase the effective sensing capacity of the biosensor (1). The polymers further comprise a dielectric polymer such as polyethylene glycol hydrogel. The dielectric polymer preserves the effective sensing capacity of the bioelectrodes (611) in the presence of the biological sample.

In a preferred embodiment of the invention, the hollow microneedles (4) are manufactured by 3D printing technology by using a photoresist based on hydrogels or conductive polymers. These hydrogels and conductive polymers act upon stimuli, either electrical, mechanical, electromagnetic or thermal stimuli. In a preferred embodiment of the invention, the hollow microneedles (4) are manufactured on an active layer, which contains the valves (62) that are activated by a bias voltage. These valves (62) control the passage of the biological sample from the first end (41) of the hollow microneedle (4) into the sensing chamber (61). For it, the valves (62) can be controlled individually or in small arrays using multiplexing techniques. In addition, the valves (62) can be fabricated in 2D on a sacrificial layer, which would then be removed using a solvent or an etching solution.

In a preferred embodiment of the invention, the valves (62) comprise polypyrrole actuators. Under the scope of interpretation of the invention, polypyrrole actuators are understood as actuators which are made of an organic polymer obtained by oxidative polymerization of pyrrole. Said actuators are good chemical stable, low voltage, moderate to large strain, and relatively high stress actuator materials. When a voltage is applied, the polypyrrole actuators undergo a deformation. Depending on the voltage applied, the polypyrrole actuators can acquire different shapes, which modify the flow of the biological sample into the sensing chamber (61). In a preferred embodiment of the invention, given three voltages (V1, V2, V3), three different polypyrrole actuactor's shapes are obtained. At V1 voltage, the polypyrrole does not undergo a deformation, so that the valve (62) remains closed, and this is interpreted as no passage of biological sample into the sensing chamber (61). However, at V2 voltage, the polypyrrole actuactor undergoes a slight deformation, so the passage of the biological sample to the sensing chamber (61) is limited. Finally, at V3 voltage, the polypyrrole actuator deforms, resulting in a maximum passage of biological sample into the detection chamber (61). In a preferred embodiment of the invention, the polyprrole actuator is connected to the drains of the field effect transistors. The use of field effect transistors to drive such polypyrrole valves (62) would facilitate their multiplexing for parallel or sequential operation and avoid the need for additional amplification or signal processing circuits. To activate the actuator, a gate voltage must be applied to activate the transistor and a variable voltage must be applied to determine the redox state of the actuator. When the voltage is - 1.3 V with respect to the reference terminal, polypyrrole actuator expands due to the influx of hydrated Na+ ions. The expansion of the conducting polymers reshapes the actuator digits to a flat state (valve closed). Then, when the source bias is increased to 0.2 V with respect to the reference terminal, polypyrrole actuator oxidizes and contracts, repelling Na+ ions and water. In this case, the microvalves bend upwards (valve opened). In another preferred embodiment of the invention the valves (62) comprise Pt thin films or magnetoelastic materials.

In a preferred embodiment of the invention, the biosensor (1) is embedded in an adhesive patch (8). With the adhesive patch (8), the biosensor (1) is attached to the subject's skin or tissue (5), either in humans or animals. In this way, the subject always wears the adhesive patch (8) penetrated its skin or tissue in a simple way. Thus, the subject does not have to prick himself every time he wants to take a measurement, which improves his life's quality. Moreover, since the patch (8) is in continuous contact with the subject's skin or tissue (5) from which the biological sample is taken, it is possible to carry out continuous chemical substances' (2) measurements.

In a preferred embodiment of the invention, the chemical substances (2) comprise hormones. The hormones are chemical messengers that travel through the bloodstream of subjects and are responsible for biological functions such as growth, metabolism, and fertility. Thus, it is quite important to have a proper hormone concentration within the bloodstream of the subject. Due to their high potency, a small deviation on the hormone concentration can cause a big impact on the subject in terms of diseases. The deviation can be understood as a deficiency or an excess of hormones concentration in the bloodstream of the subject. In a preferred embodiment the hormones are obtained from the interstitial fluid of the subject. Under the scope of interpretation of the present invention, "interstitial fluid" is understood as the liquid within the intersticium, i.e., within the space between the cells. In a preferred embodiment of the invention hormones further comprise progesterone, estradiol, luteinizing hormone and/or follicle-stimulating hormone.

In a preferred embodiment of the invention, the biosensor (1) further comprises a drug reservoir with an opening controlled by delivery means. This delivery means is adapted for delivering a drug through the opening, preferably as a function of the concentration of the chemical substances (2) present in the biological sample, as measured by the sensing unit (6) or as read from the information derived from said concentration. The drug reservoir contains drugs which are regulators of the concentration of chemical substances (2). These drugs are delivered by delivery means when the sensing unit (6) detects that the chemical substances (2) concentration is not optimal for the subject. The optimal chemical substance (2) concentration for a subject is defined by a medical specialist. In the present invention, a medical specialist will be understood as any professional with health knowledge and expertise. When the subject is an animal, the medical specialist will correspond to veterinarians or the like, while the subject is a human, the medical specialist will be a doctor, a nurse or a specialist with a similar competence.

In a preferred embodiment of the invention, the electronic circuit (612) is powered by a battery or a wireless power system. Thus, the electronic circuit (612) has energy autonomy and does not need to be connected to a grid, which allows for the biosensor's (1) portability.

A second object of the invention proposes a system (9) for monitoring biological samples applicable in clinical diagnosis comprising a biosensor (1) according to any of the preceding embodiments and a monitoring unit (10). In this embodiment, the microcontroller (7) of the biosensor (1) further comprises communication means. Said communication means is adapted for transferring the stored information in the microcontroller (7) to the monitoring unit (10). As mentioned above, the stored information corresponds to the concentration of the chemical substances (2) on the biological sample. The transfer of the stored information is preferably automatic and allows continuous and accurate monitoring of the chemical substances (2) concentration at any time. The monitoring unit (10) further comprises processing means, and display means. The processing means is configured for processing the transferred information by the communication means and generating alerts based on this information. Alerts are generated when the processed information does not conform to predefined values. These predefined values depend on the subject and have been previously defined by a medical specialist, considering which are the chemical substances (2) concentration optimal values for the subject. Thus, when the processing means detects a variation between the predefined values by the medical specialist and the processed information by the processing means, they generate an alert to warn about the variation. Said alert is displayed on the monitoring unit (10) through the display means which are also adapted for displaying the processed information. The monitoring unit (10) can be operated by the subject or by a medical specialist. Thus, the alerts generated can be visualized by both. As the monitoring of the chemical substances' (2) concentration is continuous, once a variation is detected, an alert is generated. This allows the medical specialist to adjust the treatment, e.g., modifying the drug delivery. With the system (9), personalized and effective health care is achieved.

In a preferred embodiment of the invention, the communication means is based on Bluetooth, WiFi, NFC and/or 5G protocols, or a combination thereof. With these technologies it is possible to transfer the information stored in the microcontroller (7) to the monitoring unit (10) wirelessly. Thanks to this, it is not necessary to connect the biosensor (1) directly with a cable to the monitoring unit (10) to transfer the stored information. This allows the subject wearing the biosensor (1) and the medical specialist in charge of visualizing the information processed by the processing means to be in different locations.

In a preferred embodiment of the invention, the monitoring unit (10) comprises a mobile device. Under the scope of the present invention, the expression "mobile device" will be understood as any small sized processor designed to have the capacity of processing the information transferred by the communication means from the microcontroller (7). To this end, the mobile device has processing means for processing the transferred information and display means representing the processed information and alerts. In a preferred embodiment of the invention, the mobile device may be a smartphone, a tablet, or a smartphone. The fact that the processing is done through a mobile device allows the subject or the medical specialist to visualize the monitoring at any time, since the mobile device is portable and can be carried with them.

In another preferred embodiment of the invention, the processing means comprises a Scenario Modelling framework and an artificial intelligence platform for processing the transferred information related to the chemical substances (2) in biological samples by the communication means and generating alerts based on this information. The scenario Modelling framework for remote monitoring systems (SHMS) is based on Digital Twins for Health. Under the scope of interpretation of the present invention, the expression "Digital Twins" is understood as a set of disciplines for modelling, simulating, and providing a fast deployment of different remote intelligent health monitoring scenarios with medical devices, such as Digital Twins, Internet of Things, Discrete Event Simulation, Machine Learning and Model Driven Engineering. For it, a Domain Expert creates templates for the different case studies to be treated through a Domain Web App, such as fertility hormone treatments, prostate and breast cancer and heart diseases. The Domain Web App manages all the data collected in the different previous scenarios and allows the introduction of data and behaviors from previous clinical studies. This object-oriented business logic represents the Digital Twins Templates of the domain model, such as Patient Profiles, Device Templates and Care Plan Templates. Then the medical specialist, through an App, creates a simulated SHMS scenario. For this purpose, the medical specialist creates a Digital Twins scenario which comprises a specific patient for which a Patient Twin is created, a set of specific devices, represented by Device Twins and a specific treatment by a CarePlan Twin. Afterwards, the medical specialist launches the simulation using a Discrete Event Simulation that allows to represent the events occurring in the simulated SHMS scenario, running with the Digital Twins data in a first offline version. Once the medical specialist has validated the behavior of the simulated scenario, and the events meet his needs, a transformation is applied. This transformation is based on the OOH4RIA approach for the generation of a microservice called Scenario Microservice, which allows the management of a real SHMS scenario. The microservice is composed of a business logic that contains the information of the real Devices, the real Care Plan and the Patient. Thus, once the microservice is generated, it starts collecting real data through an loT Hub (e.g., Azure loT Central) that allows connecting the logic with the loT devices in the scenario and stores the information in an operational database. Specifically, the loT Hub connects via HTTP to a smartphone acting as a gateway and via Bluetooth to the patient's set of wearable biosensors. In addition, the microservice obtains and sends patient information collected at the medical centers via standard FHIR and EHRxF APIs. At this point, the system uses the artificial intelligence platform to find patterns within the patient and device behavior data and recurrent responses to certain events, thus improving the efficiency of treatments. Then, the Digital Twins Ontology Storage repository is analyzed, which uses the industry-standard Digital Twin Domain Language (DTDL), and allows applying a coarse-grained machine learning approach, and analyzing the history of the datasets generated by the Digital Twins and by the real entities. Once the patterns are detected, the real-time DES will apply a fine-grained machine learning approach, where the machine learning algorithms are already embedded in the real SHMS scenario by means of microlearning units called learned attributes. Finally, a transfer of the data obtained by the Digital Twins and entities from the real scenario to the Digital Twins templates is performed, so that such static and dynamic information is collected in the future scenarios.

A third object of the invention proposes a method for measuring and monitoring chemical substances (2) in biological samples by using a system (9) according to any one of the previous embodiments. The method comprises the following steps;
a) placing the biosensor (1) on a subject's skin or tissue (5) so that the first end (41) of the hollow microneedle (4) penetrates the subject's skin or tissue (5);
b) taking biological samples with the first end (41) of the hollow microneedles (4); since to the microneedles (4) are hollow, the biological samples flow inside them by capillarity effect.
c) applying an electric current between the bioelectrodes (611) with the electronic circuit (612);
d) measuring the information related to the chemical substances (2) in biological samples with the sensing unit (6); once the chemical substances (2) are joined with the selective membrane, a potential variation is produced. This potential variation is measured by the electronic circuit (612) and translated into chemical substances (2) concentration.
e) storing the measured information related to the chemical substances (2) in biological samples with the microcontroller (7); the chemical substances (2) concentration obtained in the previous step c) is stored in the microcontroller (7).
f) transferring the measuring information related to the chemical substances (2) in biological samples with the communication means from the device (1) to the monitoring system (9);
g) processing the transferred information related to the chemical substances (2) in biological samples with the display means and generating alerts based on this information; when the processing means detects a variation between the predefined values by the medical specialist and the processed information by the processing means, they generate an alert to warn of the variation: and
h) displaying the alerts on the monitoring unit (10). This allows both the medical specialist and the subject to view the alerts, so that corrective actions can be taken to restore the subject's optimal chemical substances' (2) concentration.

## Claims

1. Biosensor (1) for measuring chemical substances (2) in biological samples, applicable in clinical diagnosis, **characterised in that** the biosensor (1) comprises:
- a substrate matrix (3);
- a plurality of hollow microneedles (4), adapted for penetrating a subject's skin or tissue (5) and obtaining a biological sample, wherein each of the hollow microneedles (4) comprises a first end (41) and a second end (42), wherein the first end (41) is adapted to penetrate the subject's skin or tissue (5), and wherein the second end (42) is at least partially embedded in the substrate matrix (3);
- a sensing unit (6), adapted for measuring information related to one or more chemical substances (2) in the biological sample through a potential variation, wherein the sensing unit (6) is arranged on the substrate matrix (3) and comprises one or more sensing chambers (61), wherein each of said sensing chambers (61) further comprises;
- a plurality of bioelectrodes (611), adapted for detecting the chemical substances (2), wherein the plurality of bioelectrodes (611) comprise selective membrane (6112) to the chemical substances (2) of the biological sample;
- an electronic circuit (612) adapted for applying an electric current between the bioelectrodes (611);
and wherein the potential variation takes place when one or more chemical substances (2) of the biological sample bind to the selective membrane (6112) of the bioelectrodes (611);
- a microcontroller (7), adapted for storing the information related to the chemical substances (2) in the biological sample;
wherein the sensing unit (6) comprises a plurality of valves (62) adapted for controlling the passage of the biological samples from the first end (41) of the hollow microneedle (4) into the sensing chamber (61).

2. Biosensor (1) according to the preceding claim wherein the bioelectrodes (611) comprise a semiconductor nanomaterial coupled with a plurality of electrical sensors.

3. Biosensor (1) according to the preceding claim wherein the electrical sensors are selective from: field effect transistors, electrochemical sensors or impedimetric sensors.

4. Biosensor (1) according to the preceding claim, wherein:
- the field effect transistors are embedded in one or more flexible polymer layers; and/or
- the semiconductor nanomaterials comprise carbon nanotubes, graphene, transition metal dichalcogenide monolayers or silicon nanowires.

5. Biosensor (1) according to any one of claims 1 to 4 wherein the selective membranes (6112) to the chemical substances (2) of the biological samples are based on polymers and biomolecules with high affinity for biological samples, wherein the polymers further comprise a dielectric polymer and biomolecules comprise aptamers, antibodies, nanobodies and/or enzymes.

6. Biosensor (1) according to the preceding claim wherein the hollow microneedles (4) comprise photoresist based on hydrogels or conductive polymers.

7. Biosensor (1) according to the preceding claim wherein the valves (62) comprise polypyrrole actuators.

8. Biosensor (1) according to any of the preceding claims wherein the biosensor (1) is embedded in an adhesive patch (8).

9. Biosensor (1) according to any of the preceding claims wherein the chemical substances (2) comprise hormones.

10. Biosensor (1) according to any of the preceding claims which further comprises a drug reservoir which is controlled by delivery means, wherein delivery means are adapted for delivering drug regarding the information related to the chemical substances (2) in biological samples measured by the sensing unit (6).

11. Biosensor (1) according to any of the preceding claims wherein the electronic circuit (612) is powered by a battery or a wireless power system.

12. System (9) for monitoring biological samples applicable in clinical diagnosis which comprises:
- a biosensor (1) according to any of the preceding claims wherein the microcontroller (7) further comprises communication means adapted for transferring the stored information related to the chemical substances (2) in biological samples to a monitoring unit (10);
- a monitoring unit (10), which further comprises;
- processing means, configured for processing the transferred information related to the chemical substances (2) in biological samples by the communication means and generating alerts based on this information;
- display means, adapted for displaying the processed information related to the chemical substances (2) in biological samples.

13. System (9) according to the preceding claim wherein the communication means comprise Bluetooth, WiFi, NFC and/or 5G.

14. System (9) according to any one of claims 12 to 13 wherein the monitoring unit (10) comprises a mobile device.

15. System (9) according to any one of claims 12 to 14 wherein the processing means comprises a Scenario Modelling framework and an artificial intelligence platform.
